## Europäisches Patentamt

## European Patent Office

## Office européen des brevets

(11) Publication number: **0 112 109**
**B1**

# EUROPEAN PATENT SPECIFICATION

(45) Date of publication of patent specification: **01.03.89**

(51) Int. Cl.⁴: **C 08 C 19/02, C 08 L 15/00**

(21) Application number: **83307377.8**

(22) Date of filing: **05.12.83**

(54) Sulphur-vulcanisable polymer compositions.

(30) Priority: **08.12.82 CA 417261**

(43) Date of publication of application:
**27.06.84 Bulletin 84/26**

(45) Publication of the grant of the patent:
**01.03.89 Bulletin 89/09**

(84) Designated Contracting States:
**BE DE FR GB IT NL SE**

(56) References cited:
**FR-A-2 322 878**
**FR-A-2 421 923**
**US-A-4 245 065**

(73) Proprietor: **POLYSAR LIMITED**
**Sarnia Ontario N7T 7M2 (CA)**

(72) Inventor: **Dunn, John Robert**
**1260 Marcin Road**
**Sarnia Ontario (CA)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to sulphur-vulcanisable polymer compositions comprising a sulphur vulcanising system and a hydrogenated copolymer of a conjugated diene and an α,β-unsaturated nitrile.

It is known that the carbon-carbon double bonds of polymers having such unsaturation activate vulcanisates obtained therefrom to oxidative attack. The effects of oxidising conditions on such vulcanisates have long been a problem, particularly in applications where the vulcanisates are exposed to elevated temperatures for extended periods of time.

Certain applications, such as the various hoses and seals in the engine compartment of automobiles, require vulcanised polymers having a combination of resistance to oil, to ozone and to oxidative attack in air at elevated temperatures for extended periods of time. Vulcanisates of copolymers of conjugated dienes and α,β-unsaturated nitriles, such as an acrylonitrile-butadiene copolymer, commonly known as nitrile rubber or NBR, are well known for their oil-resistance. However, they contain carbon-carbon double bond unsaturation and therefore are susceptible to oxidative attack unless subjected to special compounding procedures for the product of oxidation-resistant vulcanisates.

One obvious solution to the problem of oxidative attack is to use polymers with few or no carbon-carbon double bonds. Examples of such polymers include butyl rubber (copolymers of isobutylene and isoprene) which, typically, contains 0.5 to 3.0 mole % carbon-carbon double bond unsaturation, and ethylene-propylene copolymers which contain no such unsaturation.

Butyl rubber may be vulcanised using sulphur vulcanisation systems which include accelerators such as thiuram or dithiocarbamate compounds. Ethylene-propylene copolymers, which contain no unsaturation, cannot be vulcanised by conventional sulphur systems.

In order to reduce the amount of carbon-carbon double bond unsaturation in NBR and yet retain the copolymer's oil resistance which is thought to be provided by the nitrile functional groups in the copolymer, methods have been developed to selectively hydrogenate the carbon-carbon double bond unsaturation of NBR without hydrogenating the nitrile groups; see, for example, GB—A—1558491.

FR—A—2421923 discloses a sulphur-vulcanisable polymer composition which comprises a sulphur vulcanisation system and a partially-hydrogenated copolymer composed of 10 to 50% by weight of acrylonitrile units, 25 to 88% by weight of hydrogenated conjugated diene units and 2 to 25%, preferably 5 to 20%, by weight of conjugated diene units. Morpholine or a thiuram compound is used as an accelerator.

US—A—4245065 describes a sulphur-vulcanisable polymer composition which comprises a hydrogenated polybutadiene. The degree of carbon-carbon double bond unsaturation, in one example, is 0.9%.

US—A—4350796 discloses sulphur-vulcanisable polymer compositions which comprise a partially-hydrogenated elastomer and a vinyl chloride resin; the elastomer is derived from an unsaturated nitrile monomer and a conjugated diene monomer, and the degree of hydrogenation of the conjugated diene units is from 50 to 98 mole %. It is stated that, if the degree of hydrogenation exceeds 98 mole %, sulphur vulcanisation is then too slow to be practical.

A sulphur-vulcanisable polymer composition, according to the present invention, comprises a sulphur vulcanisation system and a partially-hydrogenated copolymer of a $C_{4-6}$ conjugated diene and a $C_{3-5}$ α,β-unsaturated nitrile, in which the copolymer contains 0.05 to 0.57 mole % carbon-carbon double bond unsaturation.

The copolymer may be selectively hydrogenated using the process described in GB—A—1558491, or other processes known to the art. The amount of carbon-carbon double bond unsaturation remaining following hydrogenation must be controlled. It has been found that, if the remaining unsaturation is not more than 0.57 mole % of the copolymer, then vulcanisates obtained from the copolymers have high resistance to ozone, good oil-resistance, and also retain elastomeric properties when exposed to oxidative conditions at elevated temperatures for extended periods of time.

Sufficient unsaturation must remain in the copolymer, to provide enough sites for reaction with the sulphur vulcanisation system, to allow a sufficient degree of cross-linking upon vulcanisation and thereby provide suitable properties of the vulcanisate. It has been found that a practical minimum amount of retained unsaturation is 0.05 mole % of the copolymer. The amount of remaining carbon-carbon double bond unsaturation in the copolymer is from 0.05 to 0.57 mole % and preferably from 0.05 to 0.1 mole %.

The $C_{4-6}$ conjugated diene used in the invention is, for example, isoprene, piperylene, dimethylene-butadiene or, preferably, butadiene. The $C_{3-5}$ nitrile used in the invention is, for example, acrylonitrile or methylacrylonitrile. Solid copolymers of butadiene and acrylonitrile are preferred. These copolymers generally contain from 15 to 50, preferably 25 to 45% by weight acrylonitrile.

The sulfur vulcanization system used herein comprises organic sulfur compounds known as accelerators. The amounts of the accelerators which are used are those which have been used in the art for the sulfur vulcanization of conventional non-hydrogenated diene-nitrile copolymers. The total weight of accelerators used is from 0.2 to 6 parts by weight per 100 parts by weight of copolymer. The accelerators are selected from thiuram, dithiocarbamate, sulfenamide, thiazole and morpholine compounds, bis-(3-(triethoxysilyl)propyl) tetrasulfide, and mixtures thereof with one another. Suitable thiuram compounds include thiuram monosulfides, disulfides, and polysulfides. Preferred thiuram monosulfides and disulfides include the tetra($C_{1-4}$-alkyl) thiuram monosulfides and disulfides. Preferred thiuram polysulfides include dipentamethylene thiuram tetrasulfide and hexasulfide. Suitable dithiocarbamate compounds include the di($C_{1-5}$-alkyl) dithiocarbamates of zinc, bismuth, nickel, cadmium, copper, lead, selenium and tellurium. Zinc dimethyl and zinc diethyl dithiocarbamate are preferred. Suitable sulfenamide compounds include the N-alkyl-, N-cycloalkyl- and N,N-dialkyl-2-benzothiazyl sulfenamides, N-oxydiethylene-2-benzothiazyl sulfenamide and the thiocarbamyl sulfenamides. N-cyclohexyl-2-benzothiazyl sulfenamide and N-oxy-diethylenedithiocarbamyl-N'-oxydiethylene sulfenamide are preferred. Suitable thiazole compounds include mercaptobenzothiazole and its derivatives. Mercaptobenzothiazole and benzothiazyl disulfide are preferred. The preferred morpholine compound is 4,4'-dithiodimorpholine. Mixtures of these compounds known in the art of sulfur vulcanization of non-hydrogenated diene-nitrile copolymers may also be used.

The sulfur vulcanization system may also include elemental sulfur. The weight of elemental sulfur, when used, is from 0.1 to 2 parts by weight per 100 parts by weight of the copolymer.

The polymer compositions of the present invention may be heated to form vulcanizates using conventional procedures well known in the art. Suitable temperatures are from 135° to 200°C, preferably from 150° to 170°C, for periods of from 2 minutes to 15 hours, preferably from 5 minutes to 30 minutes.

In the process of the present invention, the hydrogenated copolymer and the sulfur vulcanization system may be admixed in any manner known to the art for use with conventional non-hydrogenated copolymers, for example on a two-roll rubber mill or an internal mixer. Other compounding ingredients may also be mixed with the copolymer and vulcanization system in the usual manner. Such other compounding ingredients include vulcanization co-agents such as N,N'-m-phenylenedimaleimide, activators such as zinc oxide and magnesium oxide, stearic acid, antioxidants, plasticizers, processing aids, reinforcing agents, fillers, promoters and retarders in amounts well known in the art.

The polymer compositions of the present invention on vulcanization exhibit an improved combination of resistance to oils, ozone, and oxidative attack in air at elevated temperatures for extended periods of time when compared to compositions heretofore known.

The following examples illustrate the present invention but are not intended to be limiting.

Example 1

In this and succeeding examples, the same basic procedure was followed. 10 g samples of copolymer were mixed on a two-roll micro mill and compounded by addition of compounding ingredients in the conventional manner. The resulting polymer compositions were then cured at 160°C for the times shown. The physical properties of the resulting vulcanizates were then determined following ASTM standard methods as closely as possible.

Tensile properties, including tensile stress (usually known as modulus) at 100 and 300 per cent elongation and at rupture (usually known as tensile strength) and per cent elongation at rupture, were determined according to ASTM—D412—80. Hardness properties were determined using a Type A Shore durometer according to ASTM—D2240—81. Hot air aging was carried out according to ASTM—D865—81 and oil aging in ASTM Oil No. 3 according to ASTM—D471—79. Ozone resistance was determined according to ASTM—D1149—81 on Type A specimens at 40°C, 20 per cent extension and at an ozone concentration of 50 parts per 100 million parts of air by volume.

The copolymers used in this and succeeding examples are listed in Table 1. Copolymer A was a comparative solid non-hydrogenated NBR, copolymers B, C and D were comparative solid partially hydrogenated NBR's having greater than 1 mole per cent remaining unsaturation in the copolymer, and copolymers E to I were solid hydrogenated NBR's useful in the present invention. The amount of carbon-carbon double bond unsaturation in copolymers B to I was determined by proton-NMR spectroscopy and the values are believed to be accurate to ± 10 per cent of the values given. The amount of unsaturation is the mole per cent of —$CH_2$—CH=CH—$CH_2$— and/or —$CH_2$CH(CH=$CH_2$)— units in the copolymer.

Table 1

| NBR Copolymmer Designation | % by Weight Acrylonitrile | Mole Per Cent Carbon-Carbon Double Bond Unsaturation |
|---|---|---|
| A (comparative) | 34 | 65.6 |
| B (comparative) | 34 | -5.3 |
| C (comparative) | 46 | 4.4 |
| D (comparative) | 34 | 2.0 |
| E | 34 | 0.57 |
| F | 40 | 0.55 |
| G | 40 | 0.51 |
| H | 34 | 0.47 |
| I | 34 | 0.06 |

This example compares the properties of vulcanizates of the present invention prepared from Copolymers H and I with the properties of a comparative vulcanizate prepared from Copolymer A. The copolymers each were compounded by addition of the compounding ingredients listed in Table 2. Vulcanizates were prepared using different cure times as shown and the physical properties were determined. Data are given in Table 3. In this and succeeding tables, the symbol (*) indicates that the vulcanizate had deteriorated to an extent where the datum could not be determined, while the symbol (nm) indicates the value was not measured.

Table 2

| Compounding Ingredient | Amount (parts by weight per 100 parts by weight of copolymer |
|---|---|
| Sulfur | 0.4 |
| Mixed tetra($C_{1-2}$-alkyl) thiuram disulfide | 2.5 |
| N-cyclohexyl-2-benzothiazyl sulfenamide | 2.0 |
| N,N'-m-phenylenedimaleimide | 2.0 |
| Stearic acid | 0.5 |
| Zinc oxide | 5.0 |
| Magnesium oxide | 10 |
| Precipitated amorphous hydrated silica | 50 |
| Polyether polythioether plasticizer | 10 |
| Processing aid (sold under trade name TE 80 by Technical Processing Inc.) | 1.0 |
| p-Cumyldiphenyl amine | 2.0 |

4

Table 3

| Copolymer designation | A | A | H | H | I | I |
|---|---|---|---|---|---|---|
| Cure time (min.) | 7 | 17 | 14 | 24 | 14 | 24 |

Physical Properties - Unaged

| | A | A | H | H | I | I |
|---|---|---|---|---|---|---|
| Hardness | 67 | 67.5 | 69 | 70 | 70 | 70 |
| Tensile stress (MPa) | | | | | | |
| - at 100% elongation | 2.6 | 2.6 | 1.9 | 1.9 | 2.0 | 2.0 |
| - at 300% elongation | 7.1 | 7.1 | 5.4 | 4.9 | 3.7 | 3.9 |
| - at rupture | 12.9 | 12.9 | 22.1 | 21.6 | 20.1 | 19.6 |
| Elongation, % at rupture | 500 | 500 | 720 | 720 | 830 | 840 |

Physical Properties - Aged in Air at 150°C for 70 Hours

| | A | A | H | H | I | I |
|---|---|---|---|---|---|---|
| Hardness | 72 | 74 | 72 | 70 | 73 | 74 |
| Tensile stress (MPa) | | | | | | |
| - at 100% elongation | 5.4 | 5.4 | 2.0 | 3.5 | 2.9 | 2.9 |
| - at 300% elongation | 12.3 | - | 5.9 | 7.8 | 6.4 | 6.9 |
| - at rupture | 12.3 | 11.8 | 19.4 | 19.6 | 15.2 | 17.6 |
| Elongation, % at rupture | 300 | 280 | 690 | 700 | 750 | 800 |

Physical Properties - Aged in Air at 150°C for 168 Hours

| | A | A | H | H | I | I |
|---|---|---|---|---|---|---|
| Hardness | 90 | 91 | 77 | 79 | 77 | 76 |
| Tensile stress (MPa) | | | | | | |
| - at 100% elongation | * | * | 3.9 | 2.0 | 3.9 | 4.4 |
| - at 300% elongation | * | * | 9.3 | 6.9 | 8.3 | 8.3 |
| - at rupture | * | * | 17.2 | 16.2 | 14.2 | 15.2 |
| Elongation, % at rupture | 25 | 10 | 600 | 600 | 640 | 620 |

Physical Properties - Aged in Air at 150°C for 500 Hours

| | A | A | H | H | I | I |
|---|---|---|---|---|---|---|
| Hardness | nm | nm | 85 | 85 | nm | nm |
| Tensile stress (MPa) | | | | | | |
| - at 100% elongation | * | * | 6.4 | 5.9 | 5.9 | 5.9 |
| - at 300% elongation | * | * | 11.3 | 10.8 | 10.8 | 11.0 |
| - at rupture | * | * | 14.7 | 13.2 | 13.2 | 13.1 |
| Elongation, % at rupture | * | * | 520 | 500 | 500 | 450 |

Physical Properties - Aged in Air at 150°C for 1,000 Hours

| | A | A | H | H | I | I |
|---|---|---|---|---|---|---|
| Hardness | 96 | 94 | 84 | 85 | 87 | 87 |
| Tensile stress (MPa) | | | | | | |
| - at 100% elongation | * | * | 2.9 | 2.9 | 9.8 | 9.0 |
| - at 300% elongation | * | * | 12.3 | 12.7 | 13.7 | 12.7 |
| - at rupture | * | * | 13.2 | 12.7 | 13.7 | 13.7 |
| Elongation, % at rupture | * | * | 350 | 310 | 300 | 350 |

It can clearly be seen that even after 1,000 hours of aging in air at 150°C, the vulcanizates prepared from Copolymers H and I retain useful elastomeric properties whereas those from comparative Copolymer A lose most of their elastomeric properties at between 70 and 168 hours.

Example 2

This example compares properties, before and after aging, or vulcanizates obtained from polymer compositions with varying amounts of remaining carbon-carbon double bond unsaturation in the copolymer. The copolymers were compounded as in Example 1 and each was cured at 160°C for the times shown. Physical properties were then determined and data are shown in Table 4. Copolymers A to I were as described in Example 1.

It can clearly be seen from Table 4 that copolymers E to I have much superior physical properties after air aging and also much superior ozone resistance compared to copolymers A to D.

5

EP 0 112 109 B1

## Table 4

| Copolymer designation | A | B | C | D | E | F | G | H | I |
|---|---|---|---|---|---|---|---|---|---|
| Cure Time (min.) | 8.5 | 6.5 | 8.5 | 5.0 | 22.5 | 23 | 11.5 | 14 | 12.5 |

### Physical Properties - Unaged

| | A | B | C | D | E | F | G | H | I |
|---|---|---|---|---|---|---|---|---|---|
| Hardness | 64 | 71 | 71 | 70 | 67 | 72 | 68 | 69 | 71.5 |
| Tensile stress (MPa) | | | | | | | | | |
| - at 100% elongation | 2.4 | 3.7 | 2.5 | 3.0 | 1.2 | 1.5 | 2.5 | 1.9 | 1.9 |
| - at 300% elongation | 5.9 | 11.3 | 6.9 | 9.8 | 2.0 | 2.9 | 5.4 | 5.4 | 3.5 |
| - at rupture | 16.0 | 25.0 | 20.6 | 23.5 | 9.3 | 14.2 | 23.5 | 22.1 | 18.1 |
| Elongation, % at rupture | 700 | 490 | 600 | 510 | 900 | 880 | 680 | 720 | 800 |

### Physical Properties - Aged in Air at 150°C for 70 Hours

| | A | B | C | D | E | F | G | H | I |
|---|---|---|---|---|---|---|---|---|---|
| Hardness | nm | nm | nm | nm | 75 | 79 | 80 | 72 | 78 |
| Tensile stress (MPa) | | | | | | | | | |
| - at 100% elongation | 5.9 | 7.4 | 5.1 | 5.9 | 2.0 | 2.9 | 4.5 | 2.0 | 2.9 |
| - at 300% elongation | - | 14.7 | 15.2 | 17.2 | 4.4 | 5.9 | 9.3 | 5.9 | 5.9 |
| - at rupture | 12.3 | 20.0 | 20.1 | 21.6 | 16.7 | 17.2 | 20.3 | 19.4 | 16.2 |
| Elongation, % at rupture | 290 | 350 | 450 | 400 | 900 | 770 | 700 | 690 | 710 |

### Physical Properties - Aged in Air at 150°C for 168 Hours

| | A | B | C | D | E | F | G | H | I |
|---|---|---|---|---|---|---|---|---|---|
| Hardness | nm | nm | nm | nm | 78 | 82 | 81.5 | 77 | 80.5 |
| Tensile stress (MPa) | | | | | | | | | |
| - at 100% elongation | - | 14.7 | 10.3 | 9.3 | 3.7 | 3.7 | 3.5 | 3.9 | 3.5 |
| - at 300% elongation | - | - | - | 19.1 | 6.9 | 6.9 | 5.6 | 9.3 | 6.6 |
| - at rupture | 18.1 | 20.3 | 18.4 | 19.1 | 14.9 | 16.5 | 9.8 | 17.2 | 14.5 |
| Elongation, % at rupture | 30 | 200 | 270 | 300 | 820 | 630 | 625 | 600 | 650 |

(Table 4 Continued)

| Copolymer designation | A | B | C | D | E | F | G | H | I |
|---|---|---|---|---|---|---|---|---|---|
| **Physical Properties - Aged in Air at 150°C for 500 Hours** | | | | | | | | | |
| Hardness | 98 | 94.5 | 93 | 91.5 | 75 | 78 | 88 | 85 | 77 |
| Tensile stress (MPa) | | | | | | | | | |
| - at 100% elongation | * | - | - | 19.6 | 2.4 | 2.5 | 7.8 | 6.4 | 2.5 |
| - at 300% elongation | * | - | - | - | 7.4 | 9.5 | 12.3 | 11.3 | 10.3 |
| - at rupture | * | 22.1 | 20.0 | 20.6 | 12.6 | 15.0 | 13.9 | 14.7 | 13.4 |
| Elongation, % at rupture | * | 50 | 90 | 110 | 610 | 500 | 450 | 520 | 495 |
| **Physical Properties - Aged in Air at 150°C for 1,000 Hours** | | | | | | | | | |
| Hardness | nm | nm | nm | nm | nm | nm | 87.5 | 84 | nm |
| Tensile stress (MPa) | | | | | | | | | |
| - at 100% elongation | * | * | * | * | 6.5 | 6.7 | 13.7 | 2.9 | 7.8 |
| - at 300% elongation | * | * | * | * | 9.2 | 5.9 | - | 12.3 | 10.2 |
| - at rupture | * | * | 7.0 | >10.0 | 10.6 | 12.4 | 16.2 | 13.2 | 11.2 |
| Elongation, % at rupture | * | * | 20 | 30 | 530 | 480 | 220 | 350 | 400 |
| **Physical Properties - Ozone Resistance (Rating: 0=no cracks, 5=severe cracking)** | | | | | | | | | |
| - after: 24 hours | 2 | 0 | 1 | 0 | 0 | 0 | 0 | 0 | 0 |
| 48 hours | 3 | 0 | 1 | 0 | 0 | 0 | 0 | 0 | 0 |
| 72 hours | 5 | 1 | 1 | 1 | 0 | 0 | 0 | 0 | 0 |
| 96 hours | 5 | 2 | 1 | 2 | 0 | 0 | 0 | 0 | 0 |
| 120 hours | 5 | 3 | 1 | 3 | 0 | 0 | 0 | 0 | 0 |
| 144 hours | 5 | 3 | 2 | 3 | 0 | 0 | 0 | 0 | 0 |
| 168 hours | 5 | 4 | 2 | 3 | 0 | 0 | 0 | 0 | 0 |

Example 3

This example illustrates the use of different sulfur vulcanization systems containing both elemental sulfur and organic sulfur compounds to prepare vulcanizates of the present invention. Four 10 g samples of Copolymer I were compouinded with the ingredients shown in Table 5. Each was cured at 160°C for the time indicated and its physical properties determined. The data are given in Table 6.

Table 5

| Compounding Ingredient | Amount (parts by weight per 100 parts by weight of copolymer) | | | |
|---|---|---|---|---|
| Sample designation: | 5-1 | 5-2 | 5-3 | 5-4 |
| Sulfur | 1.5 | 0.5 | 0.2 | 0.4 |
| Tetramethyl thiuram monosulfide | 1.5 | --- | --- | --- |
| Benzothiazyl disulfide | 0.3 | --- | --- | --- |
| N-cyclohexyl-2-benzothiazyl sulphenamide | --- | 1.0 | 1.0 | 2.0 |
| Tetramethyl thiuram disulfide | --- | 2.0 | 1.5 | --- |
| Bis-[3-(triethyloxysilyl)propyl] tetrasulfide | --- | --- | 2.0 | --- |
| N-oxydiethylenedithiocarbamyl-N'-oxydiethylene sulfenamide | --- | --- | 2.5 | --- |
| Mixed tetra(C$_{1-2}$-alkyl) thiuram disulfide | --- | --- | --- | 2.5 |
| N,N'-m-phenylenedimaleimide | --- | --- | --- | 2.0 |
| Stearic acid | 1.0 | 1.0 | 0.5 | 0.5 |
| Zinc oxide | 5.0 | 5.0 | 5.0 | 5.0 |
| Magnesium oxide | --- | --- | 10 | 10 |
| Silica (precipitated hydrated amorphous) | 50 | 50 | 50 | 50 |
| Polyether polythioether plasticizer | 10 | 10 | 10 | 10 |
| Processing aid TE 80 | --- | --- | 1.0 | 1.0 |
| p-Cumyldiphenyl amine | 2.0 | 2.0 | 2.0 | 2.0 |

8

### Table 6

| Sample designation | 5-1 | 5-2 | 5-3 | 5-4 |
|---|---|---|---|---|
| Cure time (minutes) | 16.5 | 16.0 | 21.0 | 12.5 |

**Physical Properties - Unaged**

| | | | | |
|---|---|---|---|---|
| Hardness | 67 | 68.5 | 70.5 | 71.5 |
| Tensile stress (MPa) | | | | |
| - at 100% elongation | 1.5 | 1.5 | 1.9 | 1.9 |
| - at 300% elongation | 2.5 | 2.5 | 3.9 | 3.5 |
| - at rupture | 22.5 | 20.4 | 18.1 | 18.1 |
| Elongation, % at rupture | 860 | 830 | 820 | 800 |

**Physical Properties - Aged in Air at 150°C for 70 Hours**

| | | | | |
|---|---|---|---|---|
| Hardness | 76 | 76 | 76 | 78 |
| Tensile stress (MPa) | | | | |
| - at 100% elongation | 2.9 | 2.5 | 1.9 | 2.9 |
| - at 300% elongation | 8.8 | 6.4 | 4.9 | 5.9 |
| - at rupture | 17.2 | 18.6 | 14.7 | 16.2 |
| Elongation, % at rupture | 620 | 800 | 820 | 710 |

**Physical Properties - Aged in Air at 150°C for 168 Hours**

| | | | | |
|---|---|---|---|---|
| Hardness | 81.5 | 78 | 77 | 80.5 |
| Tensile stress (MPa) | | | | |
| - at 100% elongation | 3.9 | 3.0 | 3.0 | 3.5 |
| - at 300% elongation | 10.2 | 7.6 | 6.7 | 6.6 |
| - at rupture | 17.3 | 16.9 | 14.1 | 14.5 |
| Elongation, % at rupture | 560 | 690 | 710 | 650 |

**Physical Properties - Aged in Air at 150°C for 500 Hours**

| | | | | |
|---|---|---|---|---|
| Hardness | 78 | 74 | 78 | 77 |
| Tensile stress (MPa) | | | | |
| - at 100% elongation | 2.9 | 2.4 | 6.5 | 2.5 |
| - at 300% elongation | 13.7 | 11.6 | 11.8 | 10.3 |
| - at rupture | 17.0 | 16.5 | 14.5 | 13.4 |
| Elongation, % at rupture | 350 | 455 | 500 | 495 |

**Physical Properties - Aged in Air at 150°C for 1,000 Hours**

| | | | | |
|---|---|---|---|---|
| Hardness | nn | nn | nn | nn |
| Tensile stress (MPa) | | | | |
| - at 100% elongation | 12.7 | 9.8 | 6.9 | 7.8 |
| - at 300% elongation | - | - | 10.8 | 11.2 |
| - at rupture | 15.9 | 14.3 | 11.4 | 11.2 |
| Elongation, % at rupture | 180 | 230 | 400 | 400 |

**Physical Properties - Ozone Resistance (Rating: 0=no cracks, 5=severe cracking)**

| | | | | |
|---|---|---|---|---|
| - after 168 hours | 0 | 0 | 0 | 0 |

### Example 4

This example compares the oil resistance properties of vulcanizates of the present invention prepared from Copolymers E, F and I with those of comparative vulcanizates prepared from Copolymers A and C. Each copolymer was compounded as in Example 1. The data obtained are shown in Table 7.

## Table 7

| Copolymer designation | A | C | E | F | I |
|---|---|---|---|---|---|
| Cure time (minutes) | 7 | 11 | 22.5 | 23 | 12.5 |

Physical Properties - Aged in
ASTM Oil No. 3 at 150°C for 70 Hours

| Tensile stress (MPa) | | | | | |
|---|---|---|---|---|---|
| - at 100% elongation | 8.3 | 2.0 | 1.0 | 7.4 | 1.0 |
| - at 300% elongation | 9.3 | 5.9 | 7.4 | 5.4 | 7.4 |
| - at rupture | 9.8 | 19.1 | 6.9 | 13.2 | 8.8 |
| Elongation, % at rupture | 340 | 710 | 800 | 700 | 740 |
| % Weight Change | 8.6 | 11.9 | 19.9 | 7.8 | 21.3 |

Example 5

This example illustrates that compositions of the present invention containing carbon black may be cured using a sulfur vulcanization system. Two 10 g samples of Copolymer I were compounded with the ingredients shown in Table 8, cured at 160°C and the physical properties of the vulcanizates determined. Data are given in Table 9.

## Table 8

| Compounding Ingredient | Amount (parts by weight per 100 parts by weight of copolymer) | |
|---|---|---|
| Sample designation: | 8-1 | 8-2 |
| p-Cumyldiphenyl amine | 2.0 | 2.0 |
| Sulfur | 0.4 | 0.2 |
| Zinc Oxide | 5.0 | 5.0 |
| Stearic acid | 0.5 | 0.5 |
| Magnesium oxide | 10 | 10 |
| Carbon black ASTM Type N550 | 40 | 40 |
| Carbon black ASTM Type N762 | 50 | 50 |
| Polyether polythioether plasticizer | 20 | 20 |
| Processing aid TE 80 | 1.0 | 1.0 |
| N-cyclohexyl-2-benzothiazyl sulphenamide | 2.0 | 1.0 |
| Mixed tetra($C_{1-2}$-alkyl) thiuram disulfide | 2.5 | -- |
| Tetramethyl thiuram disulfide | -- | 1.5 |
| Bis-[3-(triethyloxysilyl)propyl] tetrasulfide | -- | 2.0 |
| N-oxydiethylenedithiocarbamyl-N'-oxydiethylene sulfenamide | -- | 2.5 |
| N,N'-m-phenylenedimaleimide | 2.0 | -- |

Table 9

| Sample Designation: | 8-1 | 8-2 |
|---|---|---|
| Cure Time (minutes): | 14.5 | 22.5 |

Physical Properties - Unaged

| Hardness | 80 | 76 |
|---|---|---|

Tensile stress (MPa)

| - at 100% elongation | 2.9 | 2.6 |
|---|---|---|
| - at 300% elongation | 5.4 | 4.7 |
| - at rupture | 9.3 | 8.2 |
| Elongation, % at rupture | 780 | 750 |

Physical Properties - Aged in Air at 150°C for 70 Hours

| Hardness | 90 | 89 |
|---|---|---|

Tensile stress (MPa)

| - at 100% elongation | 8.2 | 8.2 |
|---|---|---|
| - at rupture | 10.6 | 11.8 |
| Elongation, % at rupture | 280 | 280 |

Example 6

This example illustrates that a composition of the present invention prepared from Copolymer I may be cured using a sulfur vulcanization system containing no elemental sulfur. A 10 g sample of Copolymer I was compounded with the ingredients shown in Table 10, cured at 160° for 14 minutes and the unaged physical properties determined as shown in Table 11. A similar composition prepared from non-hydrogenated Copolymer A, compounded in an identical manner and cured at 160°C for 8.5 minutes is included for comparison.

EP 0 112 109 B1

Table 10

| Compounding Ingredient | Amount (parts by weight per 100 parts by weight of copolymer) |
|---|---|
| Poly(1,2-dihydro-2,2,4-trimethyl quinoline) | 2.0 |
| Stearic acid | 1.5 |
| Zinc oxide | 5.0 |
| Carbon black ASTM Type N550 | 40 |
| N-oxydiethylenedithiocarbamyl-N'-oxydiethylene sulfenamide | 3.0 |
| Tetramethyl thiuram disulfide | 1.0 |
| Benzothiazyl disulfide | 1.0 |
| 4,4'-Dithiodimorpholine | 2.0 |

Table 11

| Physical Propeties - Unaged | Copolymer A | Copolymer I |
|---|---|---|
| Hardness | 58 | 67 |
| Tensile stress (MPa) | | |
| - at 100% elongation | 2.9 | 2.9 |
| - at 300% elongation | 8.3 | 7.4 |
| - at rupture | 19.6 | 24.0 |
| Elongation, % at rupture | 630 | 800 |

**Claims**

1. A sulphur-vulcanizable polymer composition which comprises a sulphur vulcanisation system and a partially-hydrogenated copolymer of a $C_{4-6}$ conjugated diene and a $C_{3-5}$ α,β-unsaturated nitrile, characterised in that the copolymer contains 0.05 to 0.57 mole % carbon-carbon-double bond unsaturation.

2. A composition according to claim 1, in which the copolymer is a butadiene-acrylonitrile copolymer.

3. A composition according to claim 1 or claim 2, in which the copolymer contains from 0.05 to 0.1 mole % of the unsaturation.

4. A composition according to any preceding claim, in which the sulphur vulcanisation system comprises an accelerator selected from thiuram compounds, dithiocarbamates, sulphenamides, thiazoles, morpholines, bis(3-triethoxysilylpropyl)tetrasulphide, and mixtures thereof.

5. A composition according to claim 5, in which the sulphur vulcanisation system additionally comprises elemental sulphur.

6. A process for preparing a vulcanisate, which comprises heating a composition according to any preceding claim at from 135 to 200°C for from 2 minutes to 15 hours.

**Patentansprüche**

1. Mit Schwefel vulkanisierbare Polymer-Zusammensetzung, umfassend ein Schwefel-Vulkanisationssystem und ein partielle hydriertes Copolymer eines konjugierten $C_{4-6}$-Diens und eines α-β-ungesättigten $C_{3-5}$-Nitrils, dadurch gekennzeichnet, daß das Copolymer 0,05 bis 0,57 Mol-% Kohlenstoff-Kohlenstoff-

13

Doppelbindungs-Nichtsättigung enthält.

2. Zusammensetzung nach Anspruch 1, in der das Copolymer ein Butadien-Acrylnitril-Copolymer ist.

3. Zusammensetzung nach Anspruch 1 oder Anspruch 2, in der das Copolymer 0,05 bis 0,1 Mol-% Nichtsättigung enthält.

4. Zusammensetzung nach irgendeinem vorhergehenden Anspruch, in der das Schwefel-Vulkanisationssystem einen Accelerator umfaßt, der aus Thiuram-Verbindungen, Dithiocarbamaten, Sulfenamiden, Thiazolen, Morpholinen, Bis(3-triethoxysilylpropyl)tetrasulfid und deren Mischungen ausgewählt ist.

5. Zusammensetzung nach Anspruch 4, in der das Schwefel-Vulkanisationssystem zusätzlich elementaren Schwefel umfaßt.

6. Verfahren zur Herstellung eines Vulkanisats, umfassend das Erhitzen einer Zusammensetzung nach irgendeinem vorhergehenden Anspruch auf 135°C bis 200°C während einer Zeitspanne von 2 min bis 15 h.

**Revendications**

1. Composition de polymère vulcanisable au soufre, qui comprend un agent soufré de vulcanisation et un copolymère partiellement hydrogéné d'un diène conjugué en $C_4$ à $C_6$ et d'un nitrile en $C_3$ à $C_5$ à insaturation α,β, caractérisé en ce que le copolymère contient 0,05 à 0,57 mole % d'insaturation par doubles liaisons carbone-carbone.

2. Composition suivant la revendication 1, dans laquelle le copolymère est un copolymère butadiène-acrylonitrile.

3. Composition suivant la revendication 1 ou la revendication 2, dans laquelle le copolymère contient 0,05 à 0,01 mole % d'insaturation.

4. Composition suivant l'une quelconque des revendications précédentes, dans laquelle l'agent soufré de vulcanisation comprend un accélérateur choisi entre des thiurames, des dithiocarbamates, des sulfénamides, des thiazoles, des morpholines, le tétrasulfure de bis(3-triéthoxysilylpropyle) et leurs mélanges.

5. Composition suivant la revendication 5, dans laquelle l'agent soufré de vulcanisation comprend en outre du soufre élémentaire.

6. Procédé de préparation d'un vulcanisat, qui consiste à chauffer une composition suivant l'une quelconque des revendications précédentes à une température de 135 à 200°C pendant 2 minutes à 15 heures.